# EUROPEAN PATENT SPECIFICATION

(11) **EP 3 740 122 B1**
(45) Date of publication and mention of the grant of the patent: **24.04.2024**
(21) Application number: 19741196.0
(22) Date of filing: 16.01.2019
(51) Int. Cl.: A61B 5/00, A61B 5/145, A61B 5/1455, A61B 5/20, A61K 49/00

(54) **TRACER AGENT FOR USE IN AN IN VIVO DIAGNOSTIC METHOD FOR DIAGNOSISING A PATIENT'S RENAL FUNCTION**
TRACER-MITTEL ZUR VERWENDUNG IN EINEM IN-VIVO-DIAGNOSEVERFAHREN ZUR DIAGNOSE DER NIERENFUNKTION EINES PATIENTEN
AGENT TRACEUR UTILISABLE DANS UNE MÉTHODE DE DIAGNOSTIC IN VIVO DE LA FONCTION RÉNALE D'UN PATIENT

(30) Priority: 16.01.2018 US 201862617669 P
(43) Date of publication of application: 25.11.2020
(73) Proprietor: Mayo Foundation for Medical Education and Research, Rochester, Minnesota 55905 (US)
(72) Inventor: ROMERO, Michael F., Rochester, Minnesota 55906 (US); KASHANI, Kianoush B., Rochester, Minnesota 55902 (US); MILLER, Jordan D., Rochester, Minnesota 55902-3636 (US); ROSSANO, Adam J., Rochester, Minnesota 55901-7934 (US)
(74) Representative: Vossius & Partner Patentanwälte Rechtsanwälte mbB
(86) International application number: PCT/US2019/013784
(87) International publication number: WO 2019/143676

(56) References cited:
- WO-A1-2016/210311
- US-A1- 2003 215 391
- US-A1- 2003 236 452
- US-A1- 2006 095 102
- US-A1- 2008 281 173
- US-A1- 2010 074 401
- US-A1- 2010 087 896
- US-A1- 2013 116 512
- US-A1- 2015 010 878
- US-A1- 2015 306 486
- US-A1- 2017 112 425
- JIAGUO HUANG ET AL: "Light-Emitting Agents for Noninvasive Assessment of Kidney Function", CHEMISTRY OPEN, vol. 6, no. 4, 20 July 2017 (2017-07-20), pages 456-471, XP055729144, ISSN: 2191-1363, DOI: 10.1002/open.201700065
- EXING WANG ET AL: "A portable fiberoptic ratiometric fluorescence analyzer provides rapid point-of-care determination of glomerular filtration rate in large animals", KIDNEY INTERNATIONAL, vol. 81, no. 1, 1 January 2012 (2012-01-01), pages 112-117, XP055514970, GB ISSN: 0085-2538, DOI: 10.1038/ki.2011.294

## Description

### TECHNICAL FIELD

Aspects of the instant disclosure relate to a tracer agent for use in an in vivo diagnostic method for diagnosing a patient's renal function. In some examples, the disclosure concerns determining a glomerular filtration rate of a patient.

### BACKGROUND

Acute kidney injury (AKI) is a common complication of critical illnesses and often results in patient and institutional financial burden. Glomerular filtration rate (GFR) is considered a key indicator of kidney health, and thus, the inability to accurately and rapidly measure GFR makes real-time, renal-function assessment challenging. The current standard of care-estimated GFR (eGFR) lacks the sensitivity and accuracy needed to guide clinical decisions satisfactorily. Non-renal factors such as a patient's weight, gender, age, and racial ancestry are known to contribute to the inaccuracy of eGFR. It has also been recognized that eGFR cannot reproducibly detect subtle renal function changes. In a few sophisticated studies, iothalamate clearance is used to measure GFR, yet this method is expensive and requires hours to complete.

There remains a continuing need for an improved system and method for measuring GFR with increased accuracy, speed, consistency, convenience, and affordability. Such a system and method may serve as a viable tool to evaluate therapies aimed at regenerating renal function. For example, such a system and method may enable reliable quantification of transplanted kidney health, disease severity, progression, and drug responses in patients.

WO 2016/210311 A1 relates to methods for determining biometric indicators such as plasma volume, hematocrit and glomerular filtration rate, in mammalian subjects such as humans. The methods utilize a plurality of fluorescent tags having distinct fluorescent characteristics, which may be associated with a single static molecule, or wherein the static molecule is labeled with a fluorescent tag and a dynamic molecule is labeled with another fluorescent tag. One or more measurements of the intensities of the fluorescent emissions are taken subsequent to introduction of an injectate which contains the fluorescent tags, which can be taken using a probe or via a blood or plasma sample. Compositions and apparatuses for practicing the methods are also disclosed.

The article "Light-Emitting Agents for Noninvasive Assessment of Kidney Function" by Huang, J. and N. Gretz (Chemistry Open 2017, 6, 456-471) briefly reviews light-emitting agents, including organic fluorescence agents and inorganic renal clearable luminescent nanoparticles for the noninvasive and real-time monitoring of kidney function and disease. Moreover, some significant requirements and strategies regarding the design of ideal glomerular filtration rate agents and renal clearable nanoparticles are discussed.

The article "A portable fiberoptic ratiometric fluorescence analyzer provides rapid point-of care determination of glomerular filtration rate in large animals" by E. Wang et al. (Kidney International (2012) 81, 112-117) relates to a rapid, point-of care determination of the GFR which may provide advantages in the clinical setting. A proof of principle for such an approach is demonstrated in a pig and dogs. In both animal models, an optical fiber that delivered excitation light and collected fluorescent emissions was inserted into a peripheral vein (dog) or central venous access (pig) by means of commercial intravenous catheters. A mixture of fluorescence chimeras of a small freely filterable reporter and large non-filterable plasma volume marker were infused as a bolus, excited by light-emitting diodes, and the in vivo signals detected and quantified by photomultiplier tubes in both species in less than 60 minutes.

### SUMMARY OF THE INVENTION

The invention is defined by the features of the independent claim. Preferred embodiments are defined in the dependent claims.

### OUTLINE OF THE DISCLOSURE

Approximately 660,000 Americans suffer from kidney failure in the United States, resulting in more than $50 billion in Medicare expenditures alone. While long-term hypertension or diabetes are major contributors to chronic renal dysfunction, acute kidney injury and even renal failure is often induced in patients receiving chemotherapy for a number of cancers. Currently, there are no methods to conveniently and rapidly assess renal function in outpatient settings. Thus, it is very challenging to assess changes in renal function following medical interventions, which ultimately results in less than optimal medical management in a variety of patient populations.

While chemotherapy is a first-line treatment for many cancers, nephrotoxicity-or damage to the nephrons in the kidney-is one of the main side effects of this drug class. Thus, physicians are forced to make difficult decisions on the balance between killing cancerous cells and inducing chronic renal failure. Furthermore, the downstream effects of reducing blood pressure or optimizing diabetes management strategies on the kidney are rarely characterized. This is largely due to the testing available for the current standard of care, which involves measurement of creatinine levels-a measurement that is far too insensitive to detect early kidney damage following chemotherapy or improvements following changes in medical management strategies.

There is currently no existing hardware for clinicians to routinely, quickly, and accurately measure glomerular filtration rate in patients (current measurements, iothalimate and iohexol, require 2-4 hours and multiple blood or urine samples). All too often, this results in non-optimal medical management, the development of chronic renal failure, and a subsequent dependence on dialysis until kidney transplantation.

Kidney function is critical to maintain fluid-electrolyte homeostasis and to clear drugs and ultimately sustain life. Nephrotoxicity after transplant is a critical issue. For clinical ease, serum creatinine or cystatin C are used, but can be problematic, invalidating estimated GFR. Thus, methods to rapidly and accurately measure GFR could greatly assist in the care of patients where precise knowledge of GFR is important, including kidney donors and kidney transplant recipients.

Currently, there is a need in the art for a system and method for rapidly assessing renal function in patients. As discussed below, it is believed that the present disclosure will greatly advance patient care in both general medical assessment, intensive care unit (ICU) assessment and kidney transplant as well as oncology and cardiology practices. It is believed that the present disclosure will ultimately reduce the tremendous medical and economic burden of chronic renal failure in a number of patient populations.

Kidney function is critical to maintain fluid-electrolyte homeostasis and to clear drugs and ultimately sustain life. Nephrotoxicity after transplant is a critical issue. For clinical ease, serum creatinine or cystatin C are used, but can be problematic, invalidating estimated GFR. Thus, methods to rapidly and accurately measure GFR could greatly assist in the care of patients where precise knowledge of GFR is important, including kidney donors and kidney transplant recipients.

Current methods, such as using iothalamate to measure GFR, are labor-intensive, requiring 1-3h, and are quite inconvenient for patients. Inability to empty the bladder is a common issue requiring bladder catheters to ensure accurate collections. For transplant patients, there is not a quick way to determine GFR in living donors nor recipients after transplant. Ultimately the lack of an accurate and convenient way to measure GFR can delay recognition of acute kidney injury (AKI) or rejection, lead to inaccurate dosing of renally cleared medications, and delay institution of AKI treatment strategies.

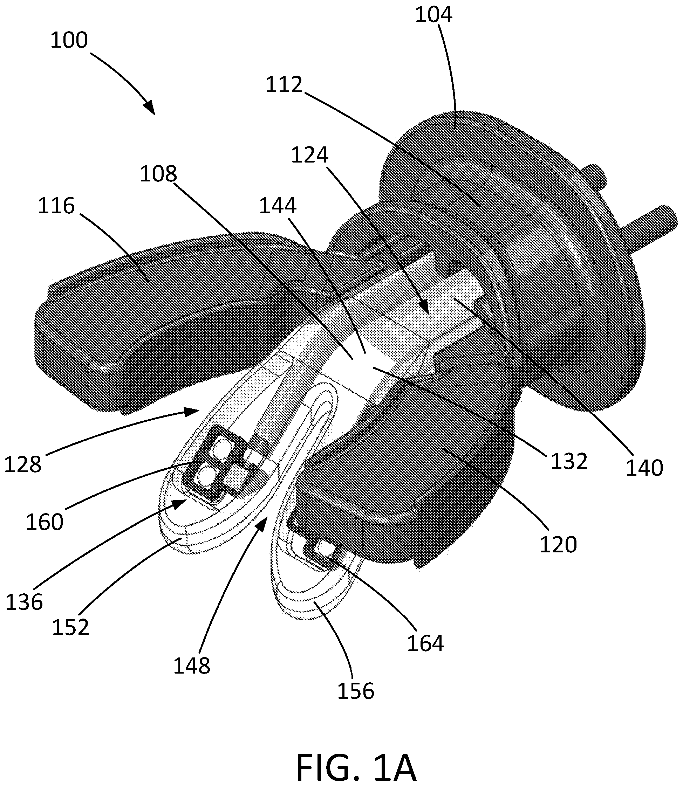

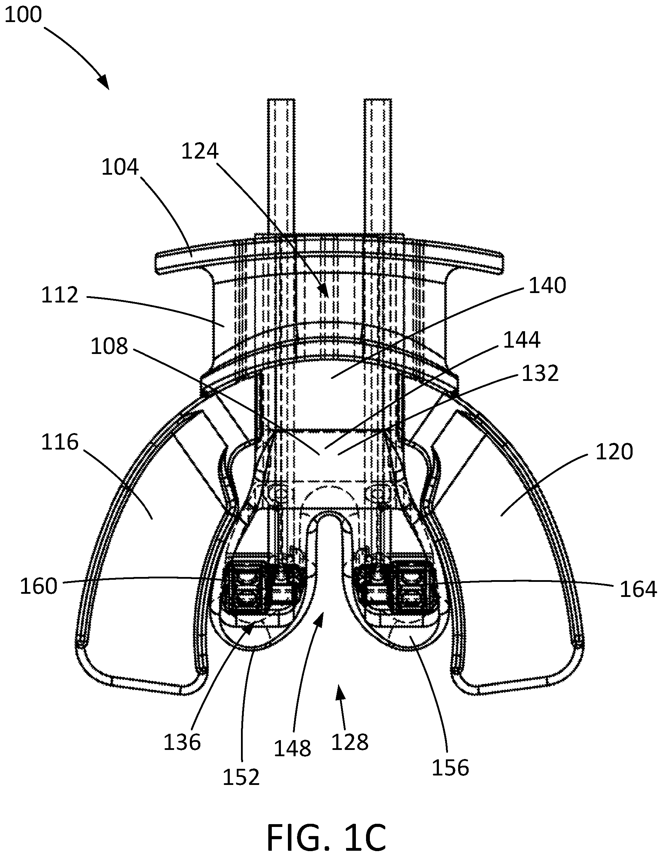

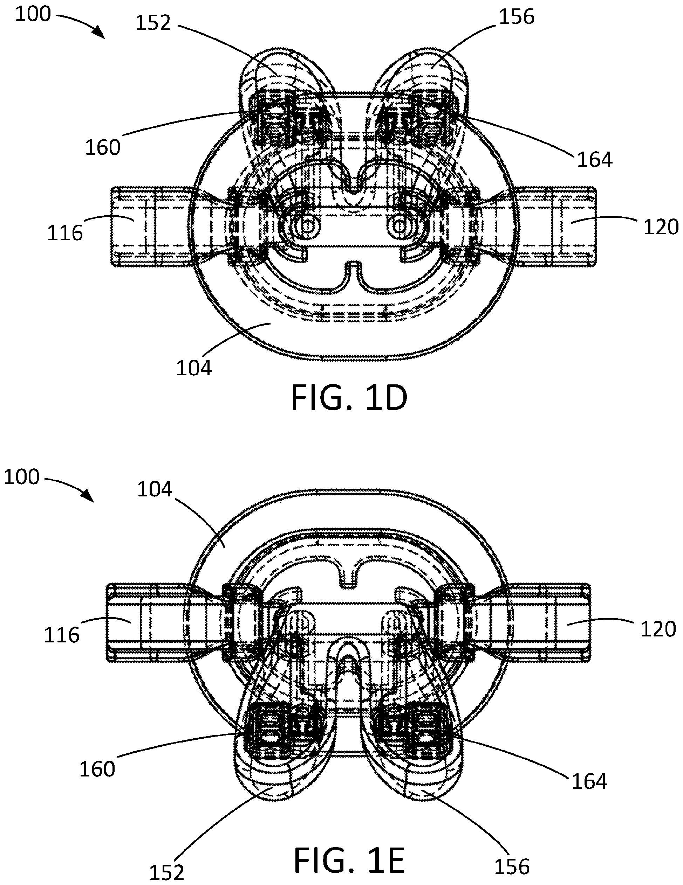

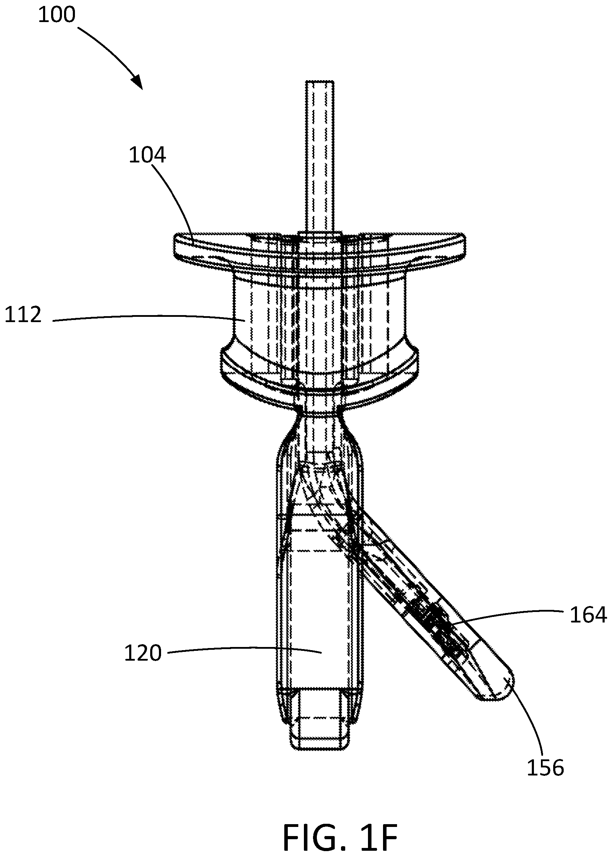

An example sublingual device (e.g., one similar to FIG. 1A) for measuring the glomerular filtration rate (GFR) of a kidney using fluorescent tracer molecules (which can simply be injected via an intravenous line) is described according to one aspect of the present disclosure. The sublingual device includes a mouthguard assembly that is configured to fit into a subject's mouth. The mouthguard assembly may include a U-shaped base member and a circular tab that protrudes from the U-shaped base member. The U-shaped base member is configured to receive a subject's upper and lower teeth, while the circular tab is configured to receive a subject's upper and lower lips. In some aspects, the circular tab is further configured with an orifice that extends through the circular tab to allow the subject to freely breathe. The mouthguard assembly is generally constructed from, for example, a deformable plastic or silicone rubber. The sublingual device further includes a tongue assembly that is configured to extend through the circular tab and to project downwards to contact a sublingual section of the subject's tongue (i.e., blood vessels positioned under the patient's tongue). The tongue assembly further includes a sensor and a prismatic reflector in electrical communication with a fiber optic cable. In some embodiments, the assembly may include four sets of sensors and prismatic reflectors. In one aspect, the sensor may comprise a LED sensor or sensor array configured to excite and measure fluorescence in the sublingual space.

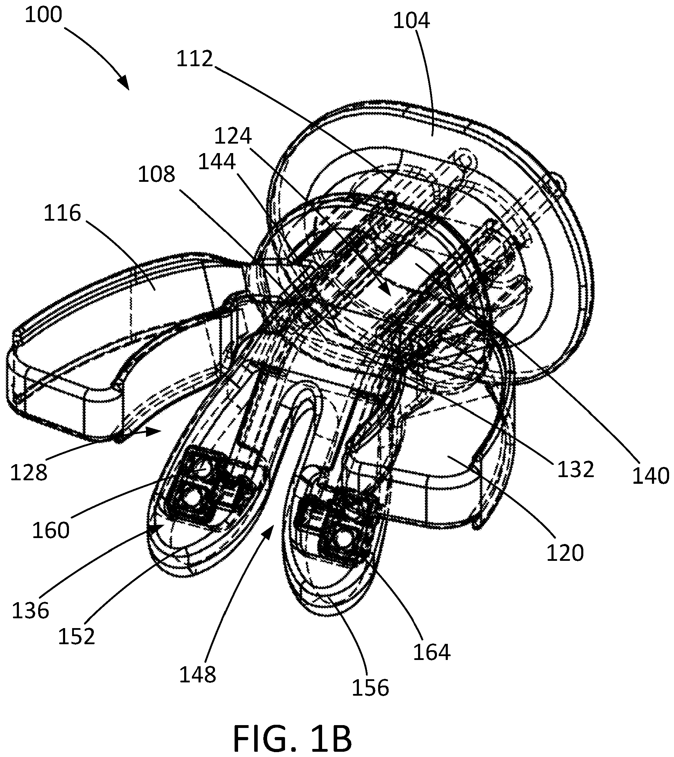

Alternative views of the sublingual device including a frontal view, a top view, a rear view, and a side view, may be similar to as shown in FIGS. 1B-1F.

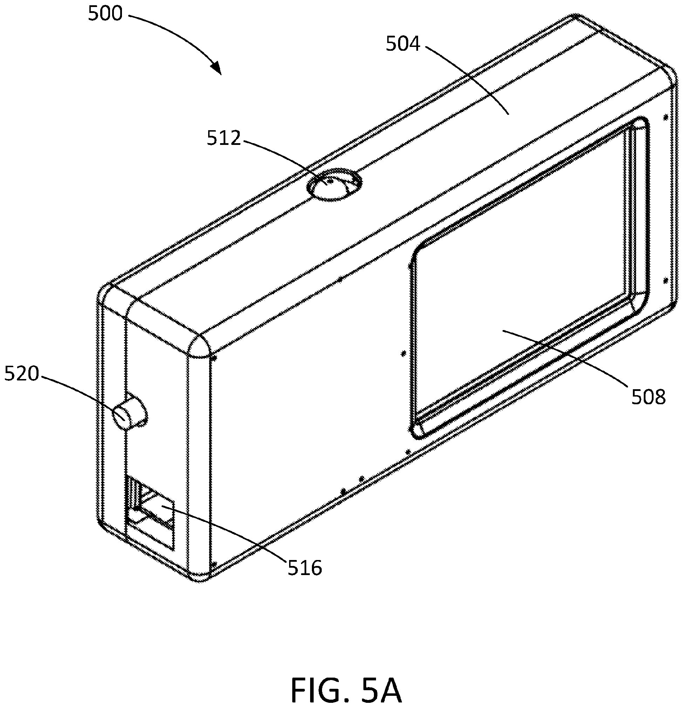

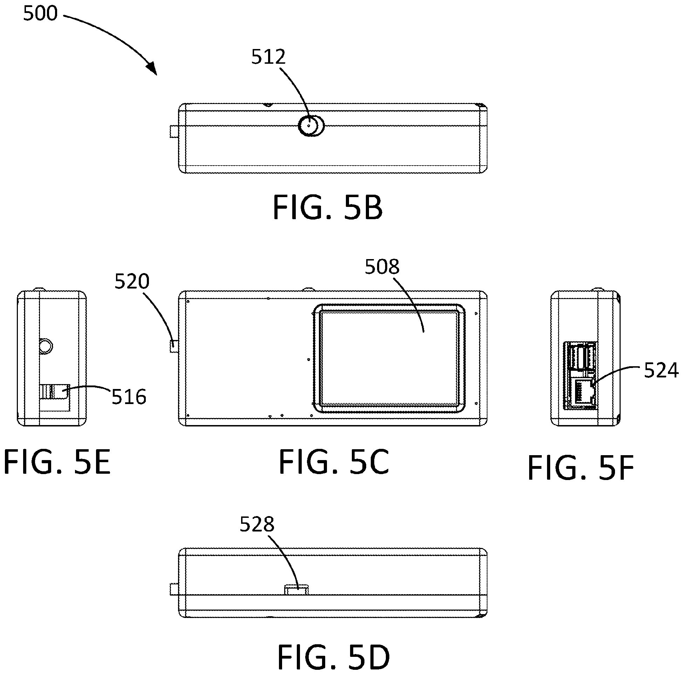

In some aspects, the sublingual device is placed in electrical communication with a spectrometer system through the fiber optic cable, (e.g., as shown in FIG. 5A). The spectrometer system may include a spectrometer, a touch screen display, and a processor A suitable spectrometer may include a visible light spectrometer. The processor may include a commercially available programmable machine running on a commercially available operating system. The spectrometer system may be powered by an internal battery and charged by a USB charger. The display provides an operator interface that facilitates entering of parameters into the spectrometer system. The sublingual device functions in response to instructions provided by the processor to operate the sensors. In one aspect, the sensors are configured to collect fluorescence data from the subject (e.g., from sublingual blood vessels), and to subsequently transfer the fluorescence data to the spectrometer using the prismatic reflector and the fiber optic cable. In some aspects, the sensors comprise LEDs of different colors that are connected to the processor by the low-voltage wire. The color for each LED is matched with the excitation wavelength of a fluorescent dye, where each dye has a different rate of filtration by the kidney. The spectrometer system may further comprise a LED control line port and a power switch.

During operation or configurable interval, the processor is programmed to illuminate a first LED color and acquire data from the spectrometer. The processor then sends signals to turn off the LED, and acquire data using the spectrometer. The preceding steps are repeated for other LED colors. In some aspects, each of the spectra are averaged to capture an average dark spectra. For each light source, the spectrum recorded is analyzed by subtracting out the averaged dark spectrum, quantifying the emission amount from the light source, and quantifying the excitation response amount, if any, from the fluorescent dye. Then, a ratio of the excitation response to the light source emissions is generated, resulting in a response ratio. For example, the ratio of the fluorescent emission of a first tracer agent (e.g., one is substantially filtered by a healthy kidney) and the fluorescent emission of a second tracer agent (e.g., one is substantially not filtered by a healthy kidney) may be used as a normalized reading of GFR, which may improve accuracy. In some embodiments, such as when only one tracer agent is used, each excitation LED is associated with an emission filter or window configured to help detect the relative fluorescent emission within a given detection, the fluorescent decay, and the fitting of the decay.

A new ratio may be generated by determining the color/dye's response ratio for a fast filtering dye to the response ratio of a slow filtering dye. Parameters of the new ratio may be fit to an exponential decay function. A glomerular filtration is then determined as the decay rate of the fitted exponential decay function. A report may be generated on the display comprising a graph of color or dye response ratios of the GFR ratio. The screen can be changed to show the raw spectra being observed by the spectrometer. Data is recorded and stored within a memory in the spectrometer system.

The following examples set forth, in detail, ways in which the sublingual device may be used or implemented and will enable one of skill in the art to more readily understand the principles thereof. The following examples are presented by way of illustration and are not meant to be limiting in any way.

In some forms, the sublingual device comprises a small computer with a touch- screen display. The computer is powered by an internal battery and is charged by a mini-USB cable. The computer connects to a mouthpiece apparatus via low-voltage electrical wire and optical cable. The optical cable is connected to a visible light spectrometer.

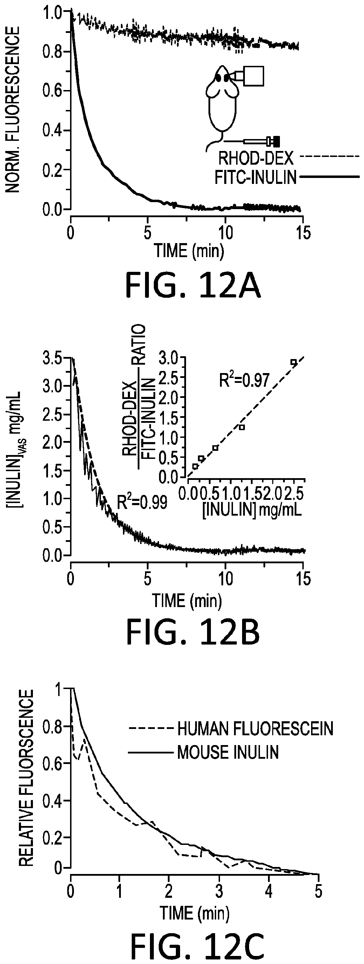

According to some examples, direct measurements of in vivo Pediatric Renal Function assessment through vascular measurements of single-bolus fluorescence markers in human and mice subjects may be similar to as shown in FIG. 12. Normalized fluorescence from retinal vasculature following sequential IV FITC-inulin (green) and Rhod-Dex (500 kD, red) using Phoenix Research Systems Micron imaging may be obtained. Traces synchronized to tail-vein injection (T = 0 min). In some embodiments, the markers are injected into mice via jugular vein injection, which may improve measurement reliability and distribution of markers. Inulin clearance based on traces of normalized fluorescence and previous calibration of Inulin/Rhod-Dex ratio in WT mouse whole blood (insert). Dashed black lines= exponential and linear (insert) curve fits to inulin concentration. Fluorescein decay extracted from patient fluorescein-angiography scan may be plotted with mouse FITC-inulin decay (clearance). To obtain the human patient data, with the help of Ophthalmology photography intersperse photos, the fluorescent decay (e.g., of AK-fluor, or fluorescein) can be visualized. After static intensity scans were collected, the images were pixel registered in Imaged so that intensity decay reflects fluorescent decay in a particular vessel.

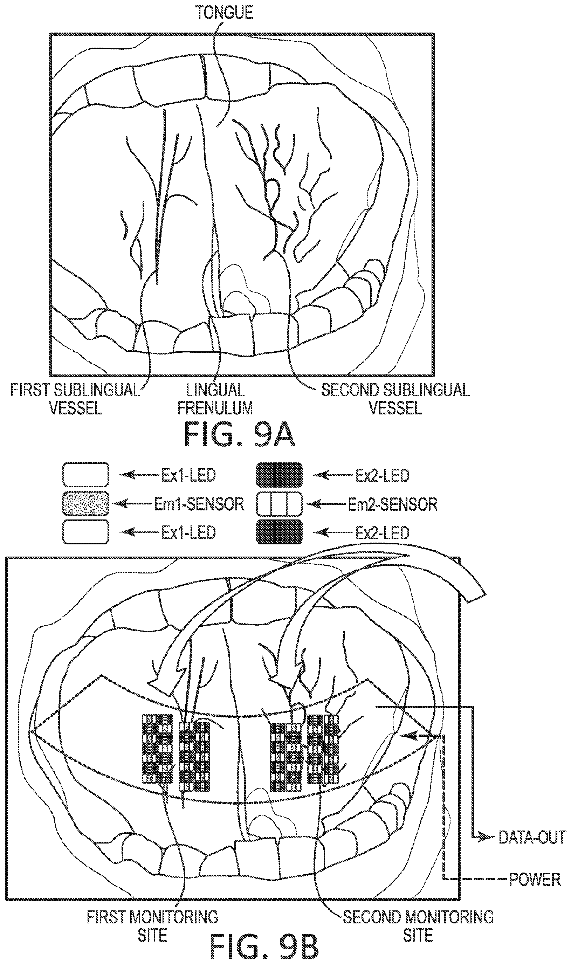

According to some examples, large sublingual vessels which can be used with surface sensors (technology already available in LEDs) are similar to as shown in FIG. 9A. A mock-up design for a flexible sensor array (see inset for details) for sublingual use may be similar to as shown in FIG. 9B. This later technique has the advantage that specialized optical equipment is not needed. As indicated, lines for power in and data out may be easily incorporated into the design.

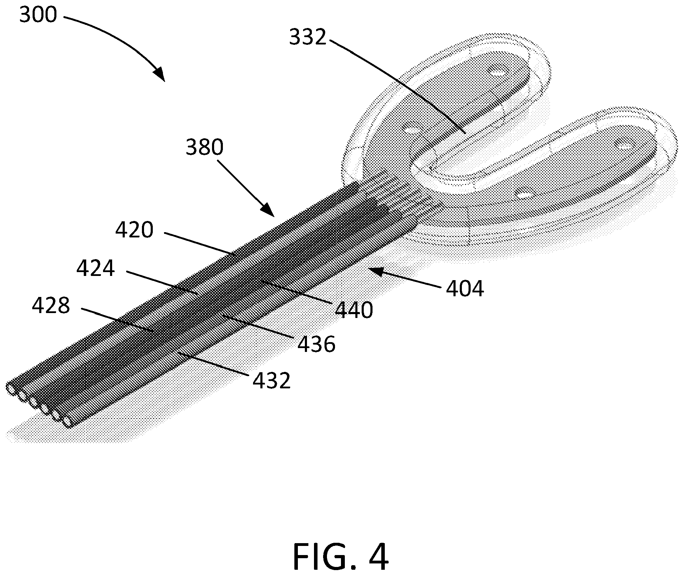

The sublingual device described (e.g., as in FIG. 4) is a non-limiting example of a sublingual GFR measurement device. During operation the device may function by switching the excitation LEDs on and off at a high rate, polling fluorescent sensors while excitation LEDs are off That is, we can excite with LED frequency A, switch off A and measure the fluorescence, excite LED frequency B, switch off B and measure the fluorescence.

Current methods of assessing renal function are time consuming, cumbersome and have inherent limitations. Developing an accurate and timely method for measuring GFR has tremendous implications for the practice of transplantation. The potential benefits of such a device are obvious in the evaluation of both living and deceased kidney donors and for post-transplant monitoring of kidney transplant recipients. In addition, it is contemplated that the sublingual device presented herein will significantly improve the care of candidates for non-renal organ transplants, particularly those being considered for a combined liver-kidney or heart-kidney transplant. Accurate measurement of GFR in patients with liver, kidney, and/or heart failure is currently quite difficult given their severity of illness. If GFR is measured accurately in such patients, clinicians could base their decision making regarding the need for a kidney transplant on real data rather than on fragmentary evidence. Such decisions have significant implications not only for the individual candidate but also for organ allocation at the local, regional and national levels.

The sublingual device of the present disclosure offers several benefits over preceding methods. For example, the sublingual device can capture fluorescein (F) & Indocyanine green (ICG) detection (F:ICG) in approximately 1-30 minutes when compared to clinically indicated urinary iothalamate clearance (1hr). Thus, the present disclosure provides an unmet need for a fast and accurate measure of renal function.

A sublingual device for measuring a glomerular filtration rate (GFR) of a kidney in a subject, the sublingual device comprising: a tongue assembly placed in contact with a sublingual section of the subject's tongue; a sensor configured within the tongue assembly, the sensor configured to measure fluorescence of a tracer molecule in the subject's tongue; and a spectrometer system in electrical communication with the sensor, the spectrometer system programmed to: determine a glomerular filtration rate of the kidney in the subject based on the fluorescence of the tracer molecule.

### BRIEF DESCRIPTION OF THE DRAWINGS

FIG. 1A shows a sublingual device, according to some examples.
FIG. 1B shows the sublingual device of FIG. 1A in a perspective view, according to some examples.
FIG. 1C shows the sublingual device of FIG. 1A in a top view, according to some examples.
FIG. 1D shows the sublingual device of FIG. 1A in a front view, according to some examples.
FIG. 1E shows the sublingual device of FIG. 1A in a rear view, according to some examples.
FIG. 1F shows the sublingual device of FIG. 1A in a side view, according to some examples.
FIG. 2 shows a tongue-engaging member of a sublingual device, according to some examples.
FIG. 3 shows a sensor assembly of a sublingual device, according to some examples.
FIG. 4 shows portions of a sublingual device, according to some examples.
FIG. 5A shows a monitoring device, according to some examples.
FIG. 5B shows the monitoring device of FIG. 5A in a top view, according to some examples.
FIG. 5C shows the monitoring device of FIG. 5A in a front view, according to some examples.
FIG. 5D shows the monitoring device of FIG. 5A in a bottom view, according to some examples.
FIG. 5E shows the monitoring device of FIG. 5A in a first side view, according to some examples.
FIG. 5F shows the monitoring device of FIG. 5A in a second side view, according to some examples.
FIG. 6 shows a detector and a control board of a monitoring device, according to some examples.
FIG. 7 shows a sublingual device connected to a monitoring device, according to some examples.
FIG. 8 depicts an illustrative method for diagnosing a patient's renal function, according to some examples.
FIG. 9A shows a sublingual vessel.
FIG. 9B shows a monitoring site encompassing the sublingual vessel of FIG. 9A, according to some examples.
FIG. 10 shows fluorescence intensities of AK-Fluor^{®} and their corresponding concentrations, according to some examples.
FIG. 11 shows a fluorescence signal recorded over a period of 10 minutes, according to some examples.
FIG. 12 shows results of a pertinent experiment for measuring GFR.
FIG. 13 illustrates a method for assessing GFR using a single fluorophore, according to some examples.
FIG. 14 illustrates a method for assessing GFR using multiple fluorophores, according to some examples.
FIG. 15 illustrates a method for assessing GFR via iGFR-guided infusion of a single fluorophore.
FIG. 16 illustrates a method for assessing GFR via a single bolus-guided infusion of a single fluorophore.
FIG. 17 illustrates a method for assessing GFR via a multiple bolus-guided infusion of a single fluorophore.
FIG. 18 illustrates a method for assessing GFR via a multiple bolus-guided infusion of a single fluorophore.

### DETAILED DESCRIPTION

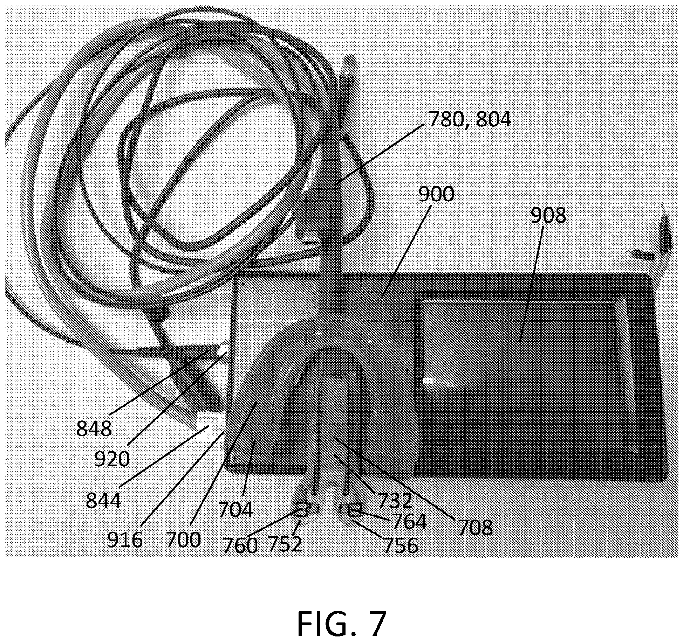

FIG. 1A shows a sublingual device 100, according to some examples. FIGS. 1B-1F provide alternative views of the sublingual device 100 by showing a perspective view, a top view, a front view, a rear view, and a side view, respectively, according to some examples. In some examples, the sublingual device 100 is for use in connection with a monitoring device (e.g., shown in FIG. 7). In various embodiments, the sublingual device 100 includes a mouth-engaging member 104 and a tongue-engaging member 108. For example, the mouth-engaging member 104 is configured to be engaged by the mouth of a patient such that the tongue-engaging member 108 extends through a mouth opening of the patient and engages with the tongue (e.g., the sublingual portion). In certain examples, the mouth-engaging member 104 includes a lip-engaging element 112, a first teeth-engaging element 116, and a second teeth-engaging element 120. In some embodiments, the lip-engaging element 112 forms or defines an entrance cavity 124 configured to receive the tongue-engaging member 108 such that the tongue-engaging member extends from one side (e.g., outside of the mouth) of the entrance cavity (or of the lip-engaging element) to another (e.g., inside of the mouth).

In various examples, the first teeth-engaging element 116 is configured to be engaged by the patient's teeth and/or the patient's first inner cheek sidewall (e.g., the left sidewall) and the second teeth-engaging element 120 is configured to be engaged by the patient's teeth and/or the patient's second inner cheek sidewall (e.g., the right sidewall). For example, the teeth-engaging elements may be gripped by teeth in the upper and lower jaws. In certain embodiments, the first teeth-engaging element 116 and the second teeth-engaging element 120 are substantially paralleled and/or mirrored. In various examples, the first teeth-engaging element 116 and the second teeth-engaging element 120 define a lingual cavity 128 into which the tongue-engaging member 108 extends. In some embodiments, the first teeth-engaging element 116 and the second teeth-engaging element 120 defines a mid-plane from which at least a portion (e.g., the distal portion) of the tongue-engaging member 108 extends below. In certain embodiments, the mouth-engaging member 104 includes silicone.

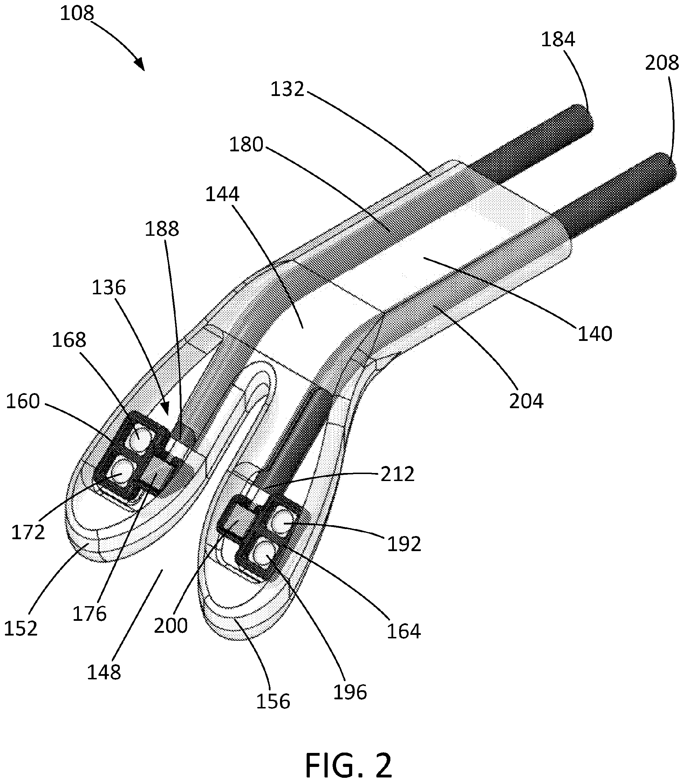

As illustrated in FIGS. 1A-1F, the tongue-engaging member 108 is configured to be coupled (e.g., slideably and/or releasably) to the mouth-engaging member 104. To describe in more detail, FIG. 2 shows the tongue-engaging member 108 of the sublingual device 100, according to some examples. In various embodiments, the tongue-engaging member 108 includes a base 132 and a monitoring assembly 136 at least partially supported by and/or embedded into the base. In some embodiments, the base 132 includes a proximal portion 140, an intermediate portion 144 distal to the proximal portion, and a distal portion 148 distal to the intermediate portion. In certain examples, the proximal portion 140 extends across the entrance cavity 124 defined by the mouth-engaging member 104 such that the intermediate portion 144 and the distal portion 148 are inside of the patient's mouth when the mouth-engaging member 104 is properly engaged (e.g., when the patient's lips engage with the lip-engaging element 112) by the patient's mouth. In certain embodiments, the intermediate portion 144 is curved such that the distal portion 148 extends below the proximal portion 140. In some examples, the distal portion includes a first lingual support 152 and a second lingual support 156. For example, the first lingual support 152 and the second lingual support 156 is substantially paralleled and/or mirrored. In various examples, the base 132 is configured to be positioned adjacent to the sublingual portion of the patient's tongue (see FIG. 9).

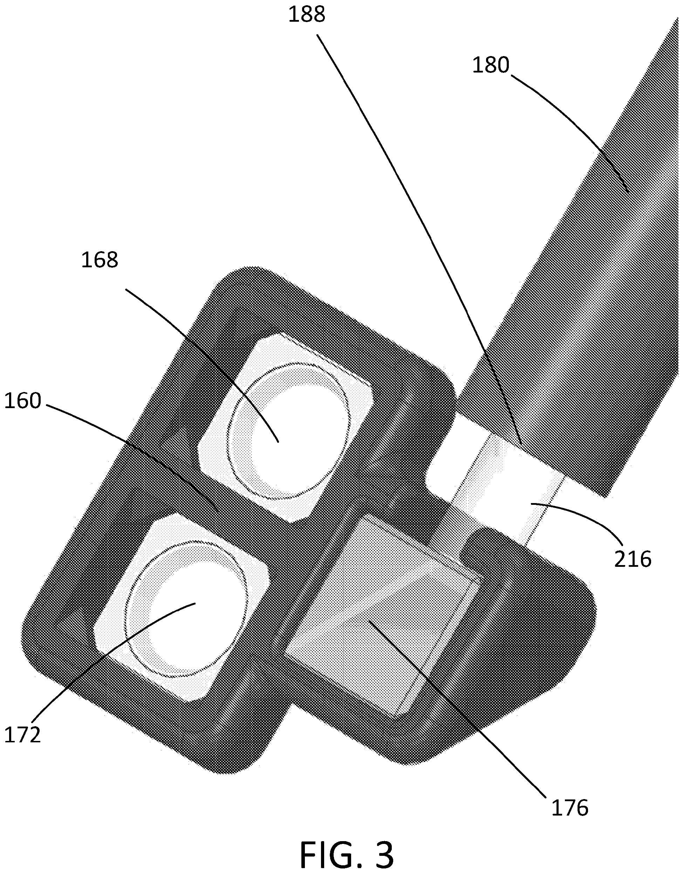

In some embodiments, the monitoring assembly 136 includes a first sensor assembly 160 and a second sensor assembly 164. FIG. 3 shows the first sensor assembly 160 in a close-up view, according to some examples. In certain embodiments, the first sensor assembly 160 and the second sensor assembly 164 are substantially similar, structurally and/or functionally. In various examples, the first sensor assembly 160 is disposed near a first side of the base 132 to which the first lingual support 152 is closer to, and the second assembly 164 is disposed near a second side of the base to which the second lingual support 156 is closer to. In some embodiments, the base 132 defines a first opening at the first lingual support 152 configured to receive at least part of the first sensor assembly 160. Similarly, in various examples, the base 132 defines a second opening at the second lingual support 152 configured to receive at least part of the second sensor assembly 164.

In various examples, the first sensor assembly 160 includes a first emitter 168, a receiver 176, a connector 180, and optionally a second emitter 172. In certain embodiments, the first emitter 168, the receiver 176, and optionally the second emitter 172 are disposed on, supported by, and/or positioned within the base 132, such as disposed on or in the first lingual support 152 at the distal portion 148. In various examples, the connector 180 extends from the distal portion 148 of the base 132 through the intermediate portion 144 and beyond the proximal portion 140 of the base 132. For example, the connector 180 includes a proximal end 184 and a distal end 188, wherein the proximal end is proximal to the proximal portion 140 of the base 132, and/or wherein the distal end is coupled or connected to the first emitter 168, the receiver 176, and optionally the second emitter 172.

Similar to the first sensor assembly 160, the second sensor assembly 164 includes a first emitter 192, a receiver 200, a connector 204, and optionally a second emitter 196, according to some examples. In certain embodiments, the first emitter 192, the receiver 200, and optionally the second emitter 196 are disposed on, supported by, and/or positioned within the base 132, such as disposed on or in the second lingual support 156 at the distal portion 148. In various examples, the connector 204 extends from the distal portion 148 of the base 132 through the intermediate portion 144 and beyond the proximal portion 140 of the base 132. For example, the connector 204 includes a proximal end 208 and a distal end 212, wherein the proximal end is proximal to the proximal portion 140 of the base 132, and/or wherein the distal end is coupled or connected to the first emitter 192, the receiver 200, and optionally the second emitter 196.

In certain examples, the base 132 is transparent at least between a first emitter and a first sublingual monitoring site and/or between a first receiver and a first sublingual monitoring site, such as when the base is positioned adjacent to the sublingual portion of the patient's tongue. In various embodiments, the first sensor assembly 160 is disposed on a first side of the base 132 and configured to be positioned at a first lateral side of the patient's lingual frenulum (see FIG. 9), such as when the base is positioned adjacent to the sublingual portion of the patient's tongue. In certain examples, the second sensor assembly 164 is disposed on a second side of the base 132 and configured to be positioned at a second lateral side of the patient's lingual frenulum (see FIG. 9), such as when the base is positioned adjacent to the sublingual portion of the patient's tongue. For example, the sensor assemblies are positioned adjacent mucus membranes encompassing blood vessels and have little or no pigment.

In certain examples, each of the emitters 168, 172, 192, and 196 is configured to emit an excitation light at an excitation wavelength. For example, the emitter 168, 172, 192, and 196 are configured to emit a first excitation light at a first excitation wavelength, a second excitation light at a second excitation wavelength, a third excitation light at a third excitation wavelength, and a fourth excitation wavelength, respectively. In certain examples, multiple of the first, second, third, and fourth excitation wavelengths are identical. For example, the first emitter 168 of the first sensor assembly 160 is configured to emit an excitation light at an excitation wavelength identical to the excitation wavelength of the excitation light the first emitter 192 of the second sensor assembly 164 is configured to emit. Similarly, in some examples, the second emitter 172 of the first sensor assembly 160 is configured to emit an excitation light at an excitation wavelength identical to the excitation wavelength of the excitation light the second emitter 196 of the second sensor assembly 164 is configured to emit. In various embodiments, the emitters are configured to emit excitation lights onto monitoring sites of the patient, such as onto sublingual monitoring sites of the sublingual portion of the patient's tongue (e.g., when the base is positioned adjacent to the sublingual portion of the patient's tongue). In certain embodiments, the emitters are configured to emit excitation lights onto excitation sites of the monitoring sites, such as sublingual excitation sites of the sublingual monitoring sites.

In certain embodiments, the first excitation wavelength can excite a first tracer agent to emit a first fluorescent light and the second excitation wavelength can excite a second tracer agent to emit a second fluorescent light. In various embodiments, the first excitation wavelength is different from the second excitation wavelength. In various embodiments, the first fluorescent light has a wavelength that is different from the wavelength of the second fluorescent light. The wavelength of the fluorescent light emitted by a tracer agent can be referred to as the fluorescent wavelength or the emission wavelength. In certain examples, the first excitation wavelength cannot excite the second tracer agent and/or the second excitation wavelength cannot excite the first tracer agent. In some examples, an emitter (emitter 168, 172, 192, and/or 196) includes a LED. In various examples, an emitter (emitter 168, 172, 192, and/or 196) includes a lens coupled to a distal end of an optical cable 216 (e.g., a fiberoptic cable), wherein the optical cable has a proximal end configured to be coupled to a source (e.g., of a monitoring device) of the excitation light. In some examples, multiple emitters are coupled to the same source for emitting excitation light of the same wavelength. In some examples, the optical cable 216 is part of the connector (e.g., connector 180 and/or 204). In certain embodiments, the connector (e.g., connector 180 and/or 204) includes an electrical connector configured to power the LED of the emitter such as by connecting the LED to a power source (e.g. of a monitoring device).

In some embodiments, the first emitter (first emitter 168 or 192) is configured to emit an excitation light at an excitation wavelength identical to the excitation wavelength of the excitation light the second emitter (second emitter 172 or 196) is configured to emit. In various examples, one or more of the emitter 168, 172, 192, 196 are configured to each emit a first excitation light at a first excitation wavelength and a second excitation light at a second excitation wavelength. For example, an emitter (emitter 168, 172, 192, and/or 196) may be configured to alternatingly emit the excitation light and the second excitation light, such as via a periodic or intermittent switching pattern. In certain embodiments, an emitter (emitter 168, 172, 192, and/or 196) includes multiple LEDs and/or is configured to be coupled to multiple sources (e.g., of a monitoring device) or to a source capable of generating multiple excitation lights at different excitation wavelengths, such as via an optical cable (e.g., optical cable 216).

In various embodiments, the receiver (receiver 176 and/or 200) is configured to receive multiple light, such as to receive multiple fluorescent light (e.g., from multiple locations and/or with multiple wavelengths), such as to receive a first fluorescent light emitted by a first tracer agent and a second fluorescent light emitted by a second tracer agent. In certain examples, the receiver (receiver 176 and/or 200) is configured to receive and direct a complex light waveform (e.g., one including the first fluorescent light and the second fluorescent light) to a detector configured to transform the complex waveform into an electrical signal (e.g., one corresponding to the first fluorescent light and the second fluorescent light) and/or into a first digital waveform (e.g., one corresponding to the first fluorescent light) and a second digital waveform (e.g., one corresponding to the second fluorescent light). In some examples, the receivers are configured to receive fluorescent light from monitoring sites of the patient, such as from the sublingual monitoring sites of the sublingual portion of the patient's tongue (e.g., when the base is positioned adjacent to the sublingual portion of the patient's tongue). In certain embodiments, the receivers are configured to receive fluorescent lights from detection sites of the monitoring sites, such as sublingual detection sites of the sublingual monitoring sites.

In some examples, the receiver (receiver 176 and/or 200) includes structures that collects light such as an optical fiber and a lens at one end of the optical fiber which directs light into the optical fiber. In various examples, the receiver (receiver 176 and/or 200) includes a detector, such as a photodetector. For example, the receiver (receiver 176 and/or 200) may include a photodetector disposed at the distal portion 148 of the base 132 and configured to transform light (e.g., fluorescent light) into digital signals for the connector (connector 180 and/or 204) to transmit to a processor (e.g., of a monitoring device). In some examples, the photodetector provides analog output. In some embodiments, the receiver (receiver 176 and/or 200) includes a directing lens (e.g., a prism) configured to direct light (e.g., fluorescent light) into a distal end of an optical cable (e.g., of connector 180 and/or 204) and out of a proximal end of the optical cable to a detector (e.g., of a monitoring device such as a spectrometer). In certain examples, the directing lens is configured to refract and/or diffract light received. In some examples, an optical cable may be an optical fiber.

In certain examples, the receiver (receiver 176 and/or 200) includes an optical filter (e.g., as part of a spectrometer) configured to filter the received light (e.g., including fluorescent light) such that the filtered light received by the detector (e.g., photodetector or spectrometer) has a single wavelength. In certain examples, the receiver (receiver 176 and/or 200) includes a digital filter (e.g., as part of a spectrometer) configured to filter the digital signal corresponding the received light (e.g., including fluorescent light) such that the filtered digital signal transmitted to a processor (e.g., of a monitoring device) corresponds to a small range or a single wavelength. In some examples, the single wavelength of the filtered light and/or that is corresponded by the filtered digital signal equals to the wavelength of a fluorescent light excited by a tracer agent (e.g., the first tracer agent).

In certain embodiments, the emitter (emitter 168, 172, 192, and/or 196) and/or the receiver (receiver 176 and/or 200) are configured to be in direct contact with the sublingual portion of a patient's tongue. In some examples, the monitoring assembly 136 includes only the first sensor assembly 160. In certain examples, each sensor assembly (sensor assembly 160 and/or 164) includes only one emitter (emitter 168 or 192) and one receiver. In some embodiments, the sensor assembly (sensor assembly 160 and/or 164) is configured to excite one or more tracer agents, such as two tracer agents, such as three tracer agents. In certain embodiments, the sensor assembly (sensor assembly 160 and/or 164) is configured to receive one fluorescent light. In some embodiments, the sensor assembly (sensor assembly 160 and/or 164) is configured to receive one or more fluorescent lights, such as two fluorescent lights, such as three fluorescent lights.

FIG. 4 shows portions of a sublingual device 300, according to some examples. For ease of viewing, some parts of the sublingual device 300 is hidden. As illustrated, the sublingual device includes a base 332, a first connector 380 coupled to the base, and a second connector 404 coupled to the base. In some examples, the first connector 380 includes a first excitation connector 420, a second excitation connector 424, and a detection connector 428. Similarly, the second connector 404 may include a first excitation connector 432, a second excitation connector 436, and a detection connector 440. A detection connector can also be referred to as a receiving connector. In various embodiments, the first connector 380 is configured to be connected to a first sensor assembly (e.g., one similar or identical to first sensor assembly 160). For example, the first excitation connector 420 may be configured to be coupled with a first emitter (e.g., one similar or identical to first emitter 168), the second excitation connector 424 may be configured to be coupled to a second emitter (e.g., one similar or identical to second emitter 172), and/or the detection connector 428 may be configured to be coupled with the receiver 176.

Similar to the first excitation connector 420, in various embodiments, the second connector 404 is configured to be connected to a second sensor assembly (e.g., one similar or identical to second sensor assembly 164). For example, the first excitation connector 432 may be configured to be coupled with a first emitter (e.g., one similar or identical to first emitter 192), the second excitation connector 436 may be configured to be coupled to a second emitter (e.g., one similar or identical to second emitter 196), and/or the detection connector 440 may be configured to be coupled with the receiver 200. In certain embodiments, one or more of the excitation connectors and the detection connectors include optical connectors configured to transmit or direct light (e.g., excitation light and/or fluorescent light). In certain examples, one or more of the excitation connectors and the detection connectors include digital connectors configured to transmit power and/or digital data.

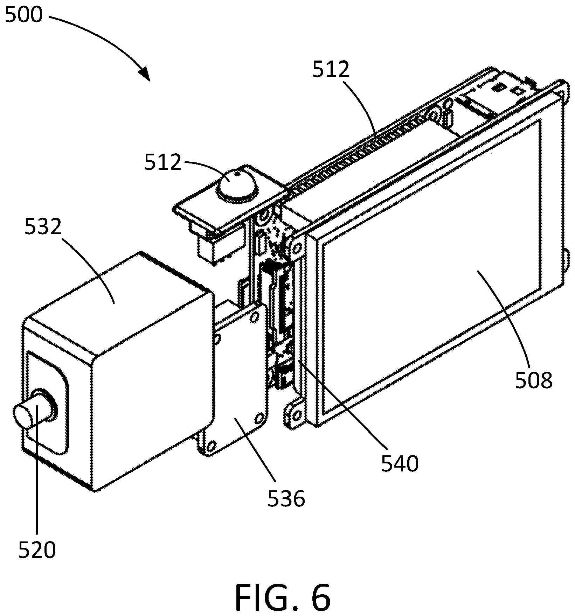

FIG. 5A shows a monitoring device 500, according to some examples. FIGS. 5B-5F provide alternative views of the monitoring device 500 by showing a top view, a front view, a bottom view, a first side view, and a second side view, respectively, according to some examples. FIG. 6 shows the monitoring device 500 unhoused, according to some examples. In various embodiments, the monitoring device 100 includes a housing 504, a display 508, a power switch 512, an excitation port 516, a detection port 520, a configuration port 524, a charging port 528, a spectrometer 532, a charging member 536, a battery 540, and a processor 544.

In some embodiments, the housing 504 houses all components of the monitoring device 100 except for the housing. In some examples, the display 508 is configured to display GFR, the concentration of one or more tracer agents, the intensity of one or more fluorescent lights (e.g., emitted by one or more tracer agents), and/or user data. In certain examples, the display 508 may be a touch screen configured for user input. In various examples, the power switch 512 (or mode switch) is configured to be manipulated (e.g., by a user) to switch between operation modes. For example, the operation modes may include monitoring device-on, monitoring device-off, excitation-on, excitation-off, detection-on, detection-off, single tracer agent mode, dual tracer agent mode, continuous excitation mode, intermittent excitation mode, continuous detection mode, intermittent detection mode, and/or a combination mode of any of the recited modes.

In various examples, the excitation port 516 is configured to be coupled to an emitter (e.g., emitter 168, 172, 192, or 196) of a sensor assembly (e.g., sensor assembly 160 or 164) to emit an excitation light. For example, the excitation port 516 may be a LED control line port configured to transmit power to a LED of a sensor assembly, such as via an electrical connector (e.g., of connector 180 or 204). In certain examples, the excitation port 516 is configured to emit an excitation light generated from a source of the monitoring device 500 such that the excitation light is transmitted from the excitation port to an emitter (e.g., emitter 168, 172, 192, or 196), such as via an optical connector (e.g., of connector 180 or 204). In some embodiments, the detection port 520 is configured to be coupled to a receiver (e.g., receiver 176 or 200) of a sensor assembly (e.g., sensor assembly 160 or 164) to receive a fluorescent light. For example, the receiving port 520 may be a fiberoptic port configured to direct received light into the spectrometer for waveform detection, processing and/or transformation.

In some examples, the configuration port 524 is configured to couple the monitoring device 500 to an external unit. For example, the external unit may be configured to process, display, monitor, record, and/or store data collected or generated by the monitoring device 500. For example, the data may include GFR, the concentration of one or more tracer agents, the intensity of one or more fluorescent lights (e.g., emitted by one or more tracer agents), and/or user data. In certain embodiments, the configuration port 524 is configured for troubleshooting and/or updating (e.g., firmware) the monitoring device 500.

In various embodiments, the charging port 528 is configured to receive a power cord such that power can be transmitted to the battery 540. For example, the charging port 528 may be a USB port configured to receive a USB cord. In certain examples, the charging member 536 is configured to receive the power from the charging port 528 and to transmit power to the battery 540. The charging member 536 may transform AC power to DC power. The charging member 536 may be a USB charger. In certain embodiments, the battery 540 is a rechargeable battery and/or rechargeable capacitor, such as a Li-ion battery, a Ni-MH battery, or a supercapacitor.

In some examples, the spectrometer 532 is configured to receive fluorescent light emitted by one or more tracer agents. For example, the spectrometer 532 is configured to generate digital data representing the wavelength and/or intensity of one or more fluorescent light. In certain embodiments, the spectrometer 532 is configured to receive and transform light signal containing a complex waveform including light signals of multiple fluorescent lights. In certain examples, the spectrometer 532 is configured to work in spectral regions near the visible spectrum. In some embodiments, spectrometer 532 is configured to work in near-IR, IR and/or far-IR spectrums, such as when the lights to be received and transformed are in one or more of such spectrums.

In various embodiments, the processor 544 is configured to generate a GFR from the digital data generated by the spectrometer 532 which represents the intensity of the fluorescent light detected by a monitoring assembly (e.g., monitoring assembly 136) of a sublingual device (e.g., sublingual device 100). For example, the processor 544 is configured to transform the digital data corresponding the intensity of the received fluorescent light into concentration of the tracer agent from which the fluorescent light emits. In certain examples, the processor 544 is also a controller configured to control one or more components of the monitoring device 500. For example, the processor 544 as a controller may be configured to control which is displayed by the display 508, when to activate the excitation port and/or the detection port. In certain examples, the processor 544 is a Raspberry Pi.

FIG. 7 shows a sublingual device 700 connected to a monitoring device 900, according to some examples. The sublingual device 700 may function and/or include components similar or identical to sublingual device 100 and/or sublingual device 300. In some examples, the sublingual device 700 includes a mouth-engaging member 704, a tongue-engaging member 708, a base 732, a first lingual support 752, a second lingual support, a first sensor assembly 760, a second sensor assembly 764, a first connector 780, and a second connector 804. In some examples, the mouth-engaging member 704 includes a teeth-engaging member configured to be engaged by the teeth of the patient. The monitoring device 900 may function and/or include components similar or identical to monitoring device 500. In various embodiments, the monitoring device 900 includes a display 908, an excitation port 916, and a detection port 920. As illustrated, the sublingual device 700 may be coupled to the monitoring device 900 via an excitation connector 844 and a detection connector 848. For example, the excitation connector 844 may couple (e.g., optically and/or electrically) the first sensor assembly 760 and the second sensor assembly 764 to the excitation port 916 such that the emitter of the sensor assemblies may emit excitation light. In certain examples, the detection connector may couple (e.g., optically and/or electrically) the first sensor assembly 760 and the second sensor assembly 764 to the detection port 920 such that the receiver of the sensor assemblies may direct fluorescent lights to the monitoring device 900.

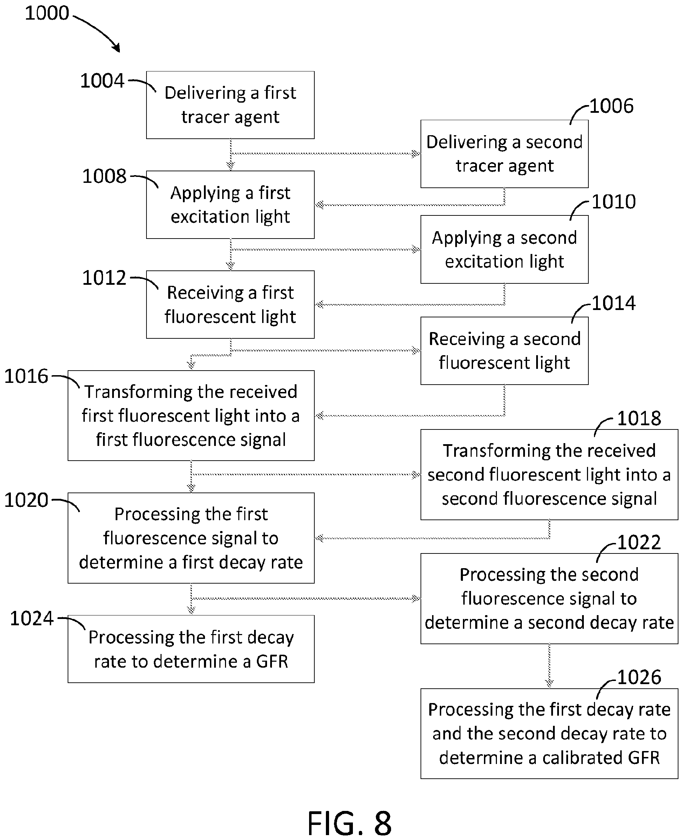

FIG. 8 depicts an illustrative method 1000 for diagnosing a patient's renal function, according to some examples. In various embodiments, the method 1000, which may be an in vivo diagnostic method, performed minimally invasively, and/or completed in under 20 min, 10 min, or 5 min. In some examples, the method 1000 includes delivering 1004 a first tracer agent, applying 1008 a first excitation light, receiving 1012 a first fluorescent light, transforming 1016 the received first fluorescent light into a first fluorescence signal, processing 1020 the first fluorescence signal to determine a first decay rate, and processing 1024 the first decay rate to determine a GFR. In certain examples, the method 1000, includes (e.g., further includes) delivering a second 1006 tracer agent, applying 1010 a second excitation light, receiving 1014 a second fluorescent light, transforming 1018 the received second fluorescent light into a second fluorescence signal, processing 1022 the second fluorescence signal to determine a second decay rate, and processing 1026 the first decay rate and the second decay rate to determine a calibrated GFR. In some examples, the GFR determined via method 1000 is referred to immediate GFR, or iGFR. In some embodiments, data representative of glomerular filtration rate of the patient's renal function may be provided based on a determined decay rate. In certain embodiments, the method 1000 may include providing data representative of glomerular filtration rate of the patient's renal function based on a determined decay rate.

In some examples, delivering 1004 a first tracer agent includes delivering a bolus of the first tracer agent into the patient's vasculature. In various embodiments, the first tracer agent is at least substantially cleared (e.g., such as cleared by more than 90% in 30 min or less) by healthy renal function and emits a first fluorescent light at a first emission wavelength in response to excitation by a first excitation light at a first excitation wavelength. In certain examples, the first tracer agent is substantially contained (e.g., with a leakage below 30%, 20%, 10%, or 5%) within the patient's vascular compartment (e.g., during the iGFR measurement procedure).

In some examples, applying 1008 a first excitation light includes applying (e.g., transmucosally or transdermally) the first excitation light to superficial (and/or visualized medium of) vasculature (or vascular beds of non-pigmented mucosal membranes, such as excluding the extracellular spaces) of the patient at a first monitoring site to excite the first tracer agent in the superficial vasculature at a first monitoring site such that the first tracer agent emits the first fluorescent light. The first monitoring site includes a first mucosal monitoring site, which includes a tympanic membrane. In various examples, the monitoring site includes a region of a membrane encompassing a vessel discernable (e.g., transmucosally or transdermally) with little to no pigment.

In some examples, receiving 1012 a first fluorescent light includes receiving (e.g., transmucosally or transdermally) the first fluorescent light emitted from the first tracer agent at the first monitoring site. In various embodiments, transforming 1016 the received first fluorescent light into a first fluorescence signal includes transforming the received first fluorescent light to produce the first fluorescence signal representative of a first fluorescence (e.g., fluorescent intensity) of the first fluorescent light (e.g., emitted by the first tracer agent). In some examples, the first decay rate (e.g., of the first fluorescent light) determined by processing 1020 the first fluorescence signal is representative of the concentration decay rate of the first tracer agent (e.g., within the patient's vasculature). In certain examples, the first decay rate is the concentration decay rate of the first tracer agent and/or the fluorescent light intensity decay rate of the first tracer agent. In various embodiments, the GFR determined by processing 1024 the first decay rate indicates level of healthiness about the patient's renal function. In some examples, the first decay rate is representative of the GFR.

In various embodiments, the first monitoring site may be a first sublingual monitoring site. In certain examples, the first monitoring site (e.g., the first mucosal monitoring site) includes non-pigmented tissue where superficial vasculature (e.g., major vessel) is discernable, such as by an optical sensor (e.g., sensor assembly 160 or 164). FIG. 9A shows a sublingual vessel and FIG. 9B shows a monitoring site (e.g., the first monitoring site) encompassing the sublingual vessel of FIG. 9A, according to some examples. The first monitoring site (e.g., the first mucosal monitoring site) includes a first mucosal excitation site and a first mucosal detection site. The first mucosal detection site is positioned downstream from the first mucosal excitation site according to the flow direction of the blood in a first mucosal vessel encompassed by the first mucosal monitoring site.

In certain examples, the first mucosal monitoring site includes a first sublingual monitoring site, the first mucosal excitation site includes a first sublingual excitation site, the first mucosal detection site includes a first sublingual detection site. The first sublingual detection site may be positioned downstream from the first sublingual excitation site according to the flow direction of the blood in a first sublingual vessel encompassed by the first sublingual monitoring site. In various embodiments, applying (e.g., transmucosally or sublingually) the first excitation light to superficial vasculature of the patient at the first monitoring site includes transmucosally applying the first excitation light to the first mucosal excitation site, and/or receiving (e.g., transmucosally or sublingually) the first fluorescent light emitted from the first tracer agent at the first monitoring site includes transmucosally receiving the first fluorescent light emitted from the first tracer agent at the first mucosal detection site. One or more additional monitoring sites may be similar to the first monitoring site, such as including a mucosal monitoring site, a sublingual monitoring site, a mucosal excitation site, a mucosal detection site, a sublingual excitation site, and/or a sublingual detection site.

In some examples, delivering 1006 a second tracer agent is performed close to delivering 1004 the first tracer agent, such as before applying 1008 the first excitation light. In various embodiments, delivering 1006 the second tracer agent includes delivering 1006 a bolus of the second tracer agent into the patient's vasculature such that the second tracer agent is present in the patient's vasculature concurrently with the first tracer agent. In certain examples, the second tracer agent is substantially not cleared (e.g., cleared by less than 30%, 20%, 10%, or 5% over 30 min) by healthy renal function and emits a second fluorescent light at a second emission wavelength in response to excitation by a second excitation light at a second excitation wavelength. In some examples, the second emission wavelength is different from the first emission wavelength. In some embodiments, the second excitation wavelength of the second excitation light is different from the first excitation wavelength of the first excitation light. In other examples, the second excitation wavelength of the second excitation light equals to the first excitation wavelength of the first excitation light.

In some examples, applying 1010 a second excitation light includes applying (e.g., transmucosally or transdermally) applying the second excitation light to superficial vasculature of the patient at a second monitoring site to excite the second tracer agent in the superficial vasculature at the second monitoring site such that the second tracer agent emits the second fluorescent light. Similar to the first monitoring site, the second monitoring site may be a second mucosal monitoring site, which may include a sublingual membrane, a tympanic membrane, an oral cavity membrane, a nasal membrane, an ocular membrane (e.g., at the lower eye lid, retinal vasculature, or ophthalmic vessel), a urinary catheter membrane, a rectal membrane, a vaginal membrane, or a pediatric superficial vessel. For example, the first monitoring site may be on a first lateral side of the patient's lingual frenulum and the second monitoring site is on a second lateral side of the patient's lingual frenulum opposite of the first monitoring site, as illustrated in FIG. 9B.

In some examples, receiving 1014 a second fluorescent light includes receiving (e.g., transmucosally or transdermally) the second fluorescent light emitted from the second tracer agent at the second monitoring site. In various embodiments, transforming 1018 the received second fluorescent light into a second fluorescence signal includes transforming the received second fluorescent light to produce the second fluorescence signal representative of a second fluorescence (e.g., fluorescent intensity) of the second fluorescent light (e.g., emitted by the second tracer agent). In some examples, the second decay rate (e.g., of the second fluorescent light) determined by processing 1022 the second fluorescence signal is representative of the concentration decay rate of the second tracer agent (e.g., within the patient's vasculature). In certain examples, the second decay rate is the concentration decay rate of the second tracer agent. In certain embodiments, the calibrated GFR determined by processing 1026 the first decay rate and the second decay rate indicates the patient's renal health. In some examples, the calibrated GFR more accurately represents the patient's renal health than the GFR determined by processing 1024 the first decay rate. For example, the calibrated GFR may substantially eliminate data error associated with tracer agent leakage from the vasculature compartment, optical artifacts from imperfect optical measurements, and/or abnormal physiologic activities.

In various embodiments, the second monitoring site may be a second sublingual monitoring site. In certain examples, the second monitoring site (e.g., the second mucosal monitoring site) includes non-pigmented tissue where superficial vasculature (e.g., major vessel) is discernable, such as by an optical sensor (e.g., sensor assembly 160 or 164). In certain examples, the second monitoring site (e.g., the second mucosal monitoring site) includes a second mucosal excitation site and a second mucosal detection site. For example, the second mucosal detection site is positioned downstream from the second mucosal excitation site according to the flow direction of the blood in a second mucosal vessel (e.g., a second sublingual vessel, see FIG. 9A) encompassed by the second mucosal monitoring site. In various embodiments, applying (e.g., transmucosally or sublingually) the second excitation light to superficial vasculature of the patient at the second monitoring site includes transmucosally applying the second excitation light to the second mucosal excitation site, and/or receiving (e.g., transmucosally or sublingually) the second fluorescent light emitted from the second tracer agent at the second monitoring site includes transmucosally receiving the second fluorescent light emitted from the second tracer agent at the second mucosal detection site. In some examples, the first monitoring site at least partially overlaps with the second monitoring site such that both the first monitoring site and the second monitoring site encompasses a vessel (e.g., a sublingual vessel) to which the first excitation light and the second excitation light are applied and from which the first fluorescent light and the second fluorescent light are emitted.

In certain examples, excitation light is applied to multiple monitoring sites and fluorescent light is received from multiple monitoring sites. For example, applying 1008 the first excitation light includes transmucosally applying the first excitation light at both lateral sides of the lingual frenulum of the patient (e.g., at both the first and the second monitoring sites), and/or receiving 1012 the first fluorescent light includes transmucosally receiving the first fluorescent light at both lateral sides of the lingual frenulum of the patient (e.g., at both the first and the second monitoring sites). In some embodiments, applying the first excitation light at both lateral sides of the lingual frenulum includes applying the first excitation light to multiple and spaced-apart locations at each of the lateral sides of the lingual frenulum. In certain examples, receiving the first fluorescent light at both lateral sides of the lingual frenulum includes receiving the first fluorescent light from multiple and spaced-apart locations at each of the lateral sides of the lingual frenulum.

In some examples, applying 1008 the first excitation light includes intermittently (e.g., periodically) applying the first excitation light. In some examples, receiving 1012 the first fluorescent light includes intermittently (e.g., periodically) receiving the first fluorescent light. In some examples, applying 1010 the second excitation light includes intermittently (e.g., periodically) applying the second excitation light. In some examples, receiving 1014 the second fluorescent light includes intermittently (e.g., periodically) receiving the second fluorescent light. In certain embodiments, intermittently applying the excitation light (e.g., the first or second excitation light) includes applying (e.g., transmucosally) excitation light (i.e., the first or second excitation light) at a frequency selected from a range of 0.0167 Hz (i.e., 1 pulse/minute) and 0.0100 Hz (e.g., 10 pulses/minute). In certain embodiments, intermittently receiving the fluorescent light (e.g., the first or second fluorescent light) includes receiving (e.g., transmucosally) fluorescent light (i.e., the first or second fluorescent light) at a frequency selected from a range of 0.0167 Hz (i.e., 1 pulse/minute) and 0.0100 Hz (e.g., 10 pulses/minute). In various embodiments, applying 1008 the first excitation light includes continuously applying the first excitation light. In some examples, receiving 1012 the first fluorescent light includes continuously receiving the first fluorescent light. In some examples, applying 1010 the second excitation light includes continuously applying the second excitation light. In some examples, receiving 1014 the second fluorescent light includes continuously receiving the second fluorescent light.

In some examples, processing 1020 the first fluorescence signal to determine the first decay rate and/or processing 1022 the second fluorescence signal to determine the second decay rate are performed over a period of less than or equal to 10 minutes (e.g., following the delivery of the bolos of the tracer agent). In various embodiments, the method 1000 further includes providing data representative of the patient's GFR. In some embodiments, the method 1000 includes at least one of storing, displaying, and transmitting GFR and/or data representative of GFR. In certain examples, the method 1000 includes recording (e.g., registering) the first fluorescence signal and/or the second fluorescence signal as a function of time.

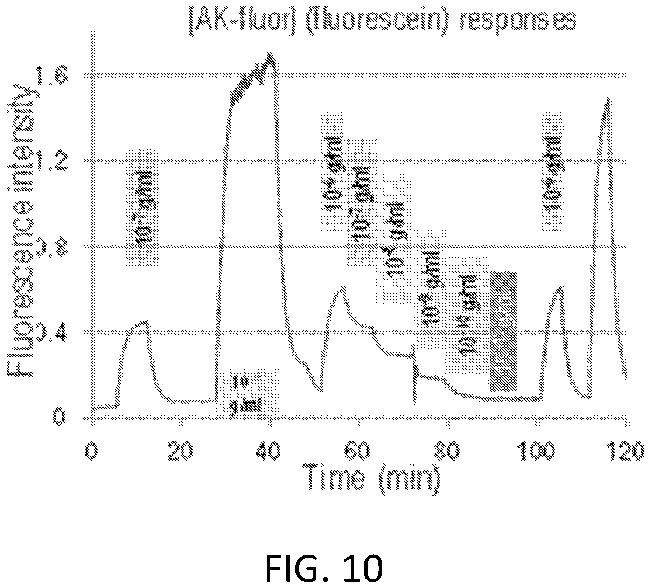

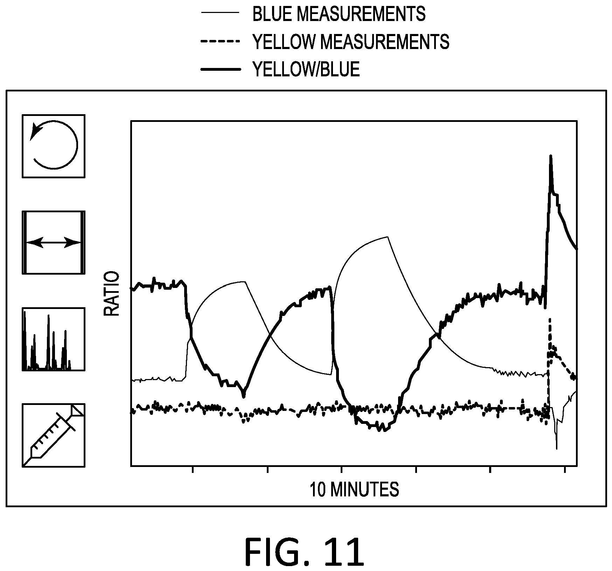

As an example, AK-Fluor^{®}, a fluorescein-based substance, may be used as a first tracer agent that is substantially cleared by healthy renal function and emits a first fluorescent light when excited by a first excitation light. FIG. 10 shows fluorescence intensities of AK-Fluor^{®} and their corresponding concentrations, according to some examples. As shown, with the use of AK-Fluor^{®}, the method of measuring GFR according to the present disclosure may provide a dynamic range from 10⁻⁵ g/ml to 10⁻¹⁰ g/ml to span 5-orders of magnitude in concentration. The relative sensitivity of concentration change of AK-Fluor^{®} as a tracer agent is high, as reflected by the corresponding fluorescence intensity shown in FIG. 10. In certain examples, the method of determining iGFR described has a response time of as fast as 0.1 sec. In some examples, a AK-Fluor^{®} of 10⁻⁷ g/ml is injected into the patient during an iGFR procedure. FIG. 11 shows a fluorescence signal recorded over a period of 10 minutes, according to some examples. As shown, in addition to recording the fluorescence signals representing the fluorescent intensities of a first tracer agent and a second tracer agent, a decay rate of the first tracer agent may also be fitted to a first exponential order. In some embodiments, a GFR can also be extrapolated, such as in real-time with the recording of the fluorescent intensities. As another example, FITC-inulin may also be used as a first tracer agent that is substantially cleared by healthy renal function and emits a first fluorescent light when excited by a first excitation light. FIG. 12 shows results of a pertinent experiment for measuring GFR. As shown in FIG. 12A, the fluorescence of FITC-inulin, as a first tracer agent is shown to decrease over a period of 15 min after injection, representing the FITC-inulin being cleared by healthy renal function. In contrast, the fluorescence of Rhod-Dextran (Rhod-Dex), as a second tracer agent is substantially not cleared over the same period of time. FIG. 12B shows inulin clearance based on measurements shown in FIG. 12A and a pre-calibrated inulin/Rhod-Dex ratio. FIG. 12C shows the fluorescein (e.g., AK-Fluor^{®}) decay extracted from a patient fluorescein angiography scan plotted with the FITC-inulin decay of FIG. 12A. It is to be understood that many alternative substances other than Rhod-Dex can be used as the second tracer agent. For example, the second tracer agent may have a molecular weight of at least 20 kDa. In some examples, multiple "first tracer agents" (e.g., inulin conjugated with different fluorescent molecules) may be injected to allow successive injections of spectrally-different "colors" to be recorded and processed by the sensor assemblies of the iGFR system (e.g., the sublingual device) described above. In some examples, a plurality of dyes may be utilized (e.g., injected and monitored) where each dye is filtered by the kidney as a tracer agent (e.g., if less than 10 kDa) or remains in the vascular volume as a reference dye (e.g., if greater than 20 kDa) during the iGFR measurement process. In certain embodiments, the filtration rates (e.g., via a healthy kidney) of at least some of the plurality of dyes are substantially similar (e.g., differs by less than 50%, 40%, 30%, 20%, or 10%). In various examples, the dye-complex to be filtered is smaller than 8 kDa. In certain examples, the dye-complex is not significantly absorbed or secreted by the renal epithelium. In some embodiments, the dye-complex is not significantly metabolized by any organ system (e.g., liver, gut).

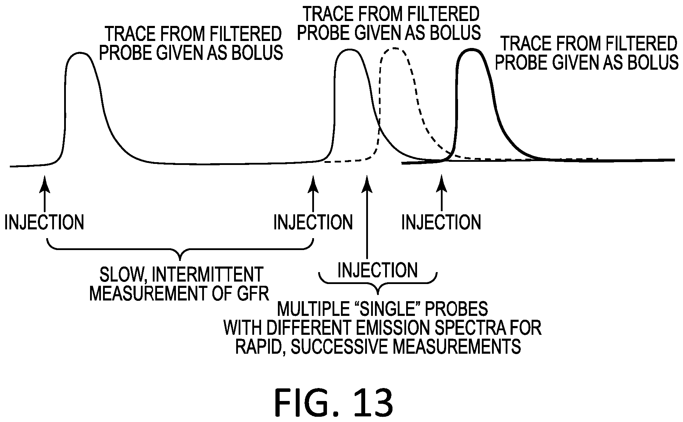

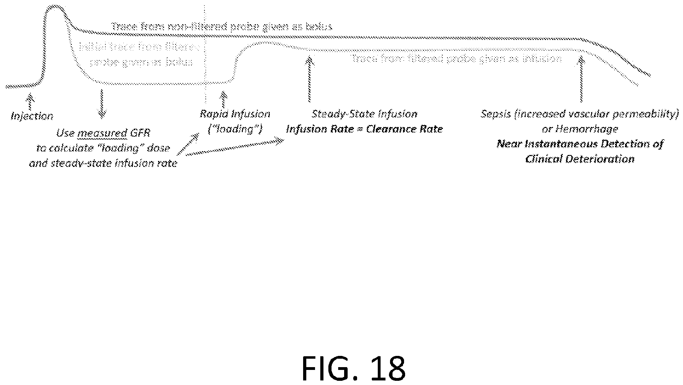

FIGS. 13-18 illustrate methods for assessing GFR with timing diagrams where concentration of one or more tracer agents are plotted against time. A tracer agent may be referred to as a probe and/or a fluorophore conjugated to a substance. FIG. 13 illustrates a method for assessing GFR using a single fluorophore, according to some examples. As shown, a single fluorophore conjugated to a substance that is rapidly and selectively filtered by the glomeruli helps with the assessment of GFR. Such a tracer agent is first injected following slow and intermittent measurement of GFR. Multiple single probes with different emission spectra may also be injected for rapid, successive measurements, which may increase GFR accuracy. In some examples, a single tracer agent that is substantially cleared by healthy renal function may be injected for measuring a standard-accuracy GFR and followed by injection of multiple tracer agents (e.g., having different fluorescent wavelengths) that are also substantially cleared by healthy renal function for measuring a high-accuracy GFR.

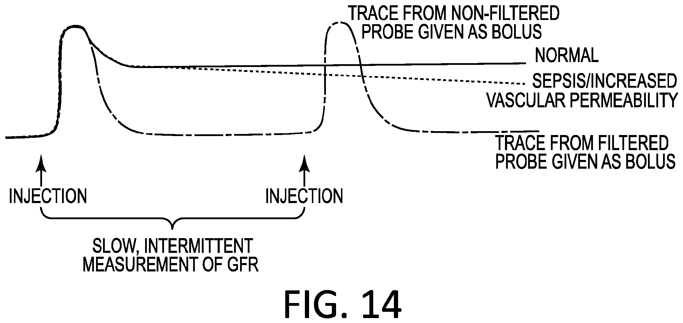

FIG. 14 illustrates a method for assessing GFR using multiple fluorophores, according to some examples. In addition to the first fluorophore conjugated to a substance that is rapidly and selectively filtered by the glomeruli to determine GFR, a second fluorophore conjugated to a substance that is not filtered by the glomeruli is used for assessment of plasma volume and interstitial leak or unwanted permeability. In some examples, the second fluorophore may help prevent overestimation of GFR and delay of intervention or treatment. In some examples, a first tracer agent (e.g., represented by the "filtered probe trace) that is substantially cleared by healthy renal function is injected concurrently with a second tracer agent (e.g., represented by the "non-filtered probe trace) that is substantially not cleared by healthy renal function, such that a calibrated GFR can be determined. In certain embodiments, the trace representing the second tracer agent may deviate from a normal condition (e.g., substantially flat), such as sloping downwards slightly over time, to indicate sepsis and/or increased vascular permeability (e.g., beyond the vascular compartment).

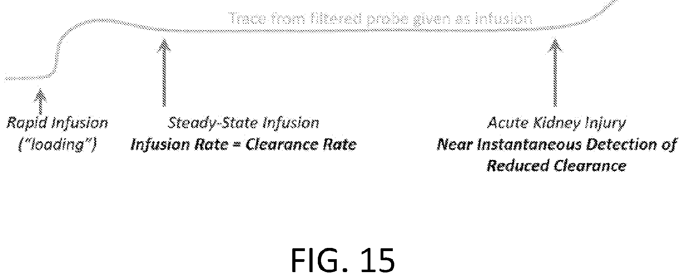

FIG. 15 illustrates a method for assessing GFR via iGFR-guided infusion of a single fluorophore. As shown, a fluorophore conjugated to a substance that is rapidly and selectively filtered by the glomeruli is used for assessment of GFR. In particularly, iGFR is measured to provide an approximation of glomerular filtration rate. In some examples, a rapid infusion or loading stage is followed by a steady-state infusion stage, which ends when a reduced clearance is detected. During the rapid infusion stage, sufficient quantity of a first tracer agent that is substantially cleared by healthy renal function may be injected such that a satisfactory concentration and/or fluorescent intensity may be detected at a monitoring site. During the steady-state infusion, more first tracer agent may be infused or injected at substantially the same rate as the clearance rate (e.g., by the kidney) such that the agent's concentration and/or fluorescent intensity remains substantially constant over time. A feedback sensing mechanism of the clearance rate may be adapted to help tune the infusion rate. In certain embodiments, by achieving such a steady-state stage, a reduction in clearance rate may be detected (e.g., near instantaneously), which may be represented by a sudden increase in concentration of the tracer agent, or in its fluorescent intensity. For example, an acute kidney injury may be detected by such a sudden deviation from the steady-state infusion equilibrium.

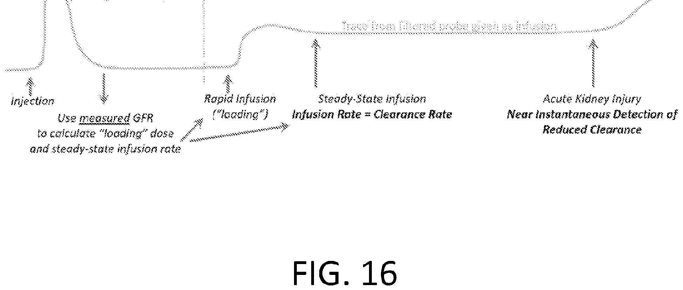

FIG. 16 illustrates a method for assessing GFR via a single bolus-guided infusion of a single fluorophore. As shown, eGFR is measured following the iGFR measurement such that the loading dose and the steady-state infusion rate are based on the iGFR result (e.g., instead of assumptions made of the patient based on weight, gender, age, and/or racial ancestry). In certain examples, the method as described in FIG. 15 may be improved by first performing an iGFR measurement such that a more accurate GFR may be used to help select a more suitable loading rate and infusion rate for the rapid infusion stage and the steady-state infusion stage. Such an improvement may help improve sensitivity of any deviation from the steady-state equilibrium, which may help detect smaller abnormalities in the patient's renal function.

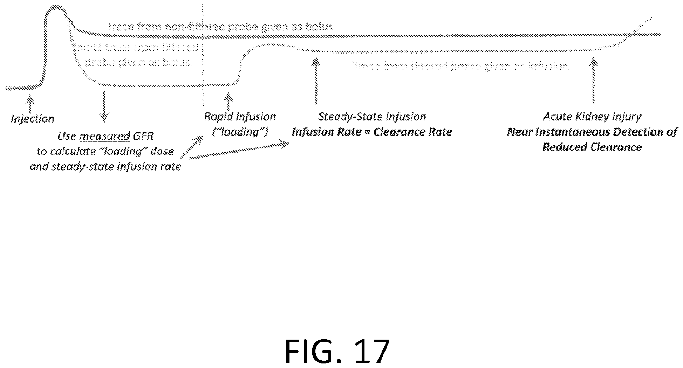

FIGS. 17-18 illustrate methods for assessing GFR via a multiple bolus-guided infusion of a single fluorophore. As shown, a first fluorophore conjugated to a substance that is rapidly and selectively filtered by the glomeruli is used for assessment of GFR. A second fluorophore conjugated to a substance that is not filtered by the glomeruli is used for assessment of plasma volume and interstitial leak/permeability. The second fluorophore may prevent overestimation of GFR, which would delay intervention/treatment. In certain examples, GFR is measured using the single bolus method, followed by infusion of the abovementioned filtered fluorophore for improved speed and accuracy. In some examples, to further increase accuracy, sensitivity, and/or speed, the method described in FIG. 16 may be modified by injecting a second tracer agent which is substantially not cleared by healthy renal function. As shown in FIGS. 17-18, when the concentration of the first tracer agent or its fluorescent intensity deviates from the steady-state equilibrium and when the concentration of the second tracer agent or its fluorescent intensity remains relatively constant, such an "event" may indicates a change in renal function. For example, a sudden reduction in clearance of the first tracer agent may indicate and acute kidney injury, as illustrated in FIG. 17. Additionally, a sudden drop in concentrations and/or intensities of both the first and the second tracer agents that ends the steady-state equilibrium may indicate sepsis which increases vascular permeability or Hemorrhage.

In some examples, alternative sensing methods may be used in place of or in addition to the fluorescence-based tracer agents, excitation means, and receiver. For example, near-IR sensors and spectrophotometers (e.g., above 800 nm) may be used with near-IR dyes to allow deeper penetration (e.g., transmucosal or transdermal), such as 1 cm to 2 cm deep. In certain examples, dermal sensor could be used to detect IR-fluorescence from major arteries or veins such as one of carotid, jugular, axillary, subclavian, femoral, and/or radial. In some examples, compounds (e.g., fluorescent compounds) for time-release delivery may be initially absorbed into the vasculature and then locked into the vascular compartment and only removed by renal filtration. For example, compounds with specifically chosen time-release coatings may be used to increase the accuracy of the iGFR method. Such compounds may be distributed through the patient's blood volume. In some embodiments, wireless technology may be adapted to enable wirelessly transmitting, displaying, storing, and/or processing of data. For example, mobile device applications may allow wireless monitoring of the iGFR experiment.

There are numerous applications of the iGFR measurement apparatus (e.g., device 100) and/or method (e.g., method 1000) described in the present disclosure. In some examples, the iGFR system and/or method may be used in space, in under-privileged areas, and/or in rural areas. In certain examples, the iGFR system and/or method may be used in outpatient clinics such as for assessment of renal function reserve in preparation of surgical procedures or use of nephrotoxic medications, evaluation of baseline kidney function for kidney donors or during the initiation of drugs that may change kidney function (e.g., ACE inhibitors, NSAIDs, contrast media), and/or when current biomarkers of GFR submission are considered suboptimal (e.g., subcopenia, obesity, liver disease, kidney disease, and/or muscle wasting). In various embodiments, the iGFR system and/or method may be used in hospitals such as for drug dosing applications where appropriate drug dosing is associated with improvement in outcome and prevention of adverse events (e.g., ones often encountered by the intensive care units), in operation room where monitoring GFR as the next vital sign (e.g., along with mean arterial pressure, respiratory rate, heart rate, and temperature) to provide additional and significant information which can potentially be used to help apply modifiable interventions to improve patient outcomes, and/or in intensive care units (ICUs) where interventions conducted routinely within ICUs could impact GFR directly or indirectly. In certain examples, early and accurate detection of such changes could provide and additional layer of information for the clinicians to avoid further changes of development of complications including AKI. For example, with intravascular volume resuscitation among patients with shock state, while volume depleted patient would increase their GFR after volume repletion, those who have volume overload additional intravascular volume expansion would most likely decrease GFR. Monitoring GFR as the next vital sign may provide useful information that could guide clinicians in the management of critically ill patients. In various examples, the iGFR system and/or method may be used for kidney failures (i.e., end-stage renal disease) applications. For example, newer continuous renal replacement therapies may include measuring renal clearance for ICU drug dosing, and intermittent hemodialysis and/or peritoneal dialysis may include measuring clearance for efficacy (e.g., instead of Kt/V). The iGFR system and/or method may also be used for research, such as for research involving uncertainties regarding the impact of interventions on kidney function. For example, the iGFR monitoring tool can be used to generate knowledge and understanding of physiology and pathophysiology of the kidney in response to different interventions and medications.

## Claims

1. A first tracer agent for use in an in vivo diagnostic method for diagnosing a patient's renal function, wherein the method comprises:
delivering (1004) a bolus of the first tracer agent into the patient's vasculature, wherein the first tracer agent is at least substantially cleared by healthy renal function and emits a first fluorescent light at a first emission wavelength in response to excitation by a first excitation light at a first excitation wavelength,
transmucosally applying (1008) the first excitation light to superficial vasculature of the patient at a first mucosal monitoring site to excite the first tracer agent in the superficial vasculature at the first mucosal monitoring site such that the first tracer agent emits the first fluorescent light;
transmucosally receiving (1012) the first fluorescent light emitted from the first tracer agent at the first mucosal monitoring site;
transforming (1016) the received first fluorescent light to produce a first fluorescence signal representative of a first fluorescence of the first fluorescent light;
processing (1020) the first fluorescence signal to determine a first decay rate of the first fluorescent light; and
processing (1024) the first decay rate of the first fluorescent light to determine a glomerular filtration rate of the patient,
wherein the first mucosal monitoring site includes a first mucosal excitation site and a first mucosal detection site, **characterised in that**, the first mucosal monitoring site includes a tympanic membrane, and **in that**
the first mucosal detection site is positioned downstream from the first mucosal excitation site according to the flow direction of the blood in a first mucosal vessel encompassed by the first mucosal monitoring site.

2. The first tracer agent for use in an in vivo diagnostic method for diagnosing a patient's renal function of claim 1, wherein processing (1020) the first fluorescence signal to determine a first decay rate of the first fluorescent light includes processing the first fluorescence signal over a period of less than or equal to 10 minutes.

3. The first tracer agent for use in an in vivo diagnostic method for diagnosing a patient's renal function of claim 1 or 2, further including recording the first fluorescence signal as a function of time.

## Patentansprüche

1. Erster Tracer zum Gebrauch in einem In-vivo-Diagnoseverfahren zur Nierenfunktionsdiagnose eines Patienten, wobei das Verfahren aufweist:
Abgeben (1004) eines Bolus des ersten Tracers in die Gefäßanordnung des Patienten, wobei der erste Tracer durch gesunde Nierenfunktion im Wesentlichen geklärt wird und ein erstes Fluoreszenzlicht mit einer ersten Emissionswellenlänge als Reaktion auf Anregung durch ein erstes Anregungslicht mit einer ersten Anregungswellenlänge emittiert,
transmukosales Applizieren (1008) des ersten Anregungslichts auf die oberflächliche Gefäßanordnung des Patienten an einer ersten mukosalen Überwachungsstelle, um den ersten Tracer in der oberflächlichen Gefäßanordnung an der ersten mukosalen Überwachungsstelle anzuregen, so dass der erste Tracer das erste Fluoreszenzlicht emittiert,
transmukosales Empfangen (1012) des ersten Fluoreszenzlichts, das vom ersten Tracer an der ersten mukosalen Überwachungsstelle emittiert wird;
Umwandeln (1016) des empfangenen ersten Fluoreszenzlichts, um ein erstes Fluoreszenzsignal als Darstellung einer ersten Fluoreszenz des ersten Fluoreszenzlichts zu erzeugen;
Verarbeiten (1020) des ersten Fluoreszenzsignals, um eine erste Abfallrate des ersten Fluoreszenzlichts zu bestimmen, und
Verarbeiten (1024) der ersten Abfallrate des ersten Fluoreszenzlichts, um eine glomeruläre Filtrationsrate des Patienten zu bestimmen,
wobei die erste mukosale Überwachungsstelle eine erste mukosale Anregungsstelle und eine erste mukosale Detektionsstelle aufweist,
**dadurch gekennzeichnet, dass** die erste mukosale Überwachungsstelle ein Trommelfell aufweist, und dadurch, dass die erste mukosale Detektionsstelle stromabwärts von der ersten mukosalen Anregungsstelle gemäß der Strömungsrichtung des Bluts in einem ersten mukosalen Gefäß positioniert ist, das durch die erste mukosale Überwachungsstelle umschlossen ist.

2. Erster Tracer zum Gebrauch in einem In-vivo-Diagnoseverfahren zur Nierenfunktionsdiagnose eines Patienten nach Anspruch 1, wobei das Verarbeiten (1020) des ersten Fluoreszenzsignals, um eine erste Abfallrate des ersten Fluoreszenzlichts zu bestimmen, aufweist: Verarbeiten des ersten Fluoreszenzsignals über eine Zeitspanne von höchstens 10 Minuten.

3. Erster Tracer zum Gebrauch in einem In-vivo-Diagnoseverfahren zur Nierenfunktionsdiagnose eines Patienten nach Anspruch 1 oder 2, das ferner aufweist: Aufzeichnen des ersten Fluoreszenzsignals als Funktion der Zeit.

## Revendications

1. Premier agent traceur destiné à être utilisé dans un procédé de diagnostic in vivo pour diagnostiquer la fonction rénale d'un patient, où le procédé comprend:
l'administration (1004) d'un bolus du premier agent traceur dans le système vasculaire du patient, où le premier agent traceur est au moins sensiblement éliminé par une fonction rénale saine et émet une première lumière fluorescente à une première longueur d'onde d'émission en réponse à une excitation par une première lumière d'excitation à une première longueur d'onde d'excitation,
l'application par voie transmuqueuse (1008) de la première lumière d'excitation au système vasculaire superficiel du patient au niveau d'un premier site de suivi muqueux pour exciter le premier agent traceur dans le système vasculaire superficiel au niveau du premier site de suivi muqueux de telle sorte que le premier agent traceur émet la première lumière fluorescente;
la réception par voie transmuqueuse (1012) de la première lumière fluorescente émise par le premier agent traceur au niveau du premier site de suivi muqueux;
la transformation (1016) de la première lumière fluorescente reçue pour produire un premier signal de fluorescence représentatif d'une première fluorescence de la première lumière fluorescente;
le traitement (1020) du premier signal de fluorescence pour déterminer un premier taux de décroissance de la première lumière fluorescente; et
le traitement (1024) du premier taux de décroissance de la première lumière fluorescente pour déterminer un taux de filtration glomérulaire du patient,
où le premier site de suivi muqueux inclut un premier site d'excitation muqueux et un premier site de détection muqueux, **caractérisé en ce que** le premier site de suivi muqueux inclut une membrane tympanique, et **en ce que** le premier site de détection muqueux est positionné en aval du premier site d'excitation muqueux selon le sens d'écoulement du sang dans un premier vaisseau muqueux englobé par le premier site de suivi muqueux.

2. Premier agent traceur destiné à être utilisé dans un procédé de diagnostic in vivo pour diagnostiquer la fonction rénale d'un patient selon la revendication 1, où le traitement (1020) du premier signal de fluorescence pour déterminer un premier taux de décroissance de la première lumière fluorescente inclut le traitement du premier signal de fluorescence sur une période inférieure ou égale à 10 minutes.

3. Premier agent traceur destiné à être utilisé dans un procédé de diagnostic in vivo pour diagnostiquer la fonction rénale d'un patient selon la revendication 1 ou 2, incluant en outre l'enregistrement du premier signal de fluorescence en fonction du temps.
